(19) Europäisches Patentamt
European Patent Office
Office européen des brevets

(11) Veröffentlichungsnummer : **0 185 962**
**B1**

(12) # EUROPÄISCHE PATENTSCHRIFT

(45) Veröffentlichungstag der Patentschrift : **25.05.88**

(51) Int. Cl.⁴ : **C 07 D233/58**

(21) Anmeldenummer : **85115160.5**

(22) Anmeldetag : **29.11.85**

(54) **Verfahren zur Herstellung von Imidazolen.**

(30) Priorität : **05.12.84 DE 3444337**

(43) Veröffentlichungstag der Anmeldung : **02.07.86 Patentblatt 86/27**

(45) Bekanntmachung des Hinweises auf die Patenterteilung : **25.05.88 Patentblatt 88/21**

(84) Benannte Vertragsstaaten : **BE CH DE FR GB IT LI NL**

(56) Entgegenhaltungen :
**EP-A- 0 018 568**
**EP-A- 0 024 533**
**EP-A- 0 026 908**
**EP-A- 0 039 828**
**DE-A- 2 360 175**

(73) Patentinhaber : **BASF Aktiengesellschaft**
**Carl-Bosch-Strasse 38**
**D-6700 Ludwigshafen (DE)**

(72) Erfinder : **Mesch, Walter, Dr.**
**Richinesstrasse 11**
**D-6700 Ludwigshafen (DE)**

**EP 0 185 962 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein verbessertes Verfahren zur Herstellung von Imidazolen durch Umsetzung einer α, β-Dicarbonylverbindung mit einem Aldehyd und Ammoniak nach Radziszewski.

Die Herstellung von Imidazol und in 2-, 4- und 5-Stellung substituierten Imidazolen nach Radziszewski (vgl. Radziszewski, Chem. Ber. 15, 1493 (1883) ; Ullmanns Enzyklopädie der techn. Chemie, 4. Auflage, Band 13, Seite 173 (1977) und K. Hofmann : The Chemistry of Heterocyclic Compounds, Imidazole and Its Derivatives, Part I, Seite 33, 1953) verläuft nach folgendem Reaktionsschema :

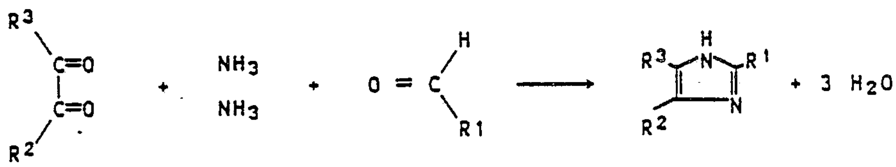

mit $R^1$, $R^2$, $R^3$ = Alkyl, Aryl oder H.

Ein erheblicher Nachteil dieser Reaktion liegt in den hohen Anteilen dabei anfallender, nicht flüchtiger, harzig-viskoser Nebenprodukte, die die Ausbeute mindern, die Abtrennung der reinen Imidazole erschweren und entsorgt werden müssen.

Der Erfindung lag daher die Aufgabe zugrunde, die Herstellung von Imidazolen in der Weise zu verbessern, daß der Anteil a Nebenprodukten stark zurückgedrängt wird.

Es wurde nun gefunden, daß sich Imidazole durch Umsetzung einer α, β-Dicarbonylverbindung mit einem Aldehyd und Ammoniak besonders vorteilhaft herstellen lassen, wenn man das nach der Umsetzung anfallende rohe Reaktionsgemisch direkt oder nach Wechsel des Lösungsmittels katalytisch hydriert.

Als α, β-Dicarbonylkomponente kommen Verbindungen der allgemeinen Formel

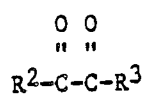

in Betracht, in denen die Reste $R^2$ und $R^3$ gleich oder verschieden sind und einen verzweigten oder unverzweigten Alkylrest mit 1 bis 10, insbesondere 1 bis 5 Kohlenstoffatomen, einen Arylrest mit 6 bis 10 Kohlenstoffatomen oder Wasserstoff bedeuten, also z. B. Ethylglyoxal, Methylglyoxal, Butandion-(2,3) oder Benzil. Für das erfindungsgemäße Verfahren besonders vorteilhaft ist die Verwendung von Glyoxal, das zweckmäßig in Form einer wäßrigen z. B. 40 %igen Lösung eingesetzt wird.

Für die Kondensation in Betracht kommende Aldehyde sind solche der allgemeinen Formel $R^1CHO$, in der der Rest $R^1$ einen verzweigten oder unverzweigten Alkylrest mit 1 bis 10, insbesondere 1 bis 5 Kohlenstoffatomen bedeutet oder einen Arylrest mit 6 bis 12 Kohlenstoffatomen, also z. B. eine Methyl-, Ethyl-, Propyl-, Isopropyl-, Butyl-, Isobutyl-, tert.-Butyl-, Pentyl-, Isopentyl- oder eine Phenylgruppe. Besonders starke Ausbeuteerhöhungen an Imidazol werden erzielt, wenn man die Radziszewski-Synthese mit Formaldehyd durchführt. Der Formaldehyd wird zweckmäßig in Form einer wäßrigen z. B. 30 bis 40 %igen Lösung verwendet.

Die Umsetzung der drei Reaktionspartner erfolgt in an sich bekannter Weise, wobei die Reaktionspartner α,β-Dicarbonylverbindung, Aldehyd und Ammoniak im allgemeinen in einem Molverhältnis von 1 : 1,0 bis 2,5 : 1,5 bis 5 zusammengegeben werden. Vorteilhaft ist ein Molverhältnis von 1 : 1,0 bis 1,5 : 2,0 bis 3,5. Die Umsetzung wird in der Regel bei Temperaturen von 40 bis 100, insbesondere 50 bis 80 °C innerhalb von 2 bis 6 Stunden durchgeführt.

Das nach der Umsetzung erhaltene Reaktionsgemisch wird in der Regel direkt zur Hydrierung weiterbenutzt. Häufig ist es vorteilhaft, vor der Hydrierung einen Wechsel des Lösungsmittels vorzunehmen, indem man z. B. das wäßrige Reaktionsgemisch mit einem organischen Lösungsmittel extrahiert. Für die Extraktion verwendet man zweckmäßigerweise ein Lösungsmittel, das möglichst wenig wasserlöslich ist, die Umsetzungsprodukte wie Imidazole oder deren Vorstufen gut löst und sich im nachfolgenden Hydrierungsschritt inert verhält. Hier kommen insbesondere längerkettige Amine wie Dibutylamin, Ether wie Diethylether, Methyl-tert.-butylether und vor allem höhere Alkohole mit 4 bis 8 Kohlenstoffatomen in Betracht, also z. B. Isobutanol, Butanol, Pentanol, Isopentanol oder Cyclohexanol.

Zweckmäßig verwendet man das Lösungsmittel in einer Menge von 20 bis 300, vorzugsweise von 50 bis 200 Gew.%, bezogen auf die α, β-Dicarbonylverbindung.

Die Umsetzung mit Wasserstoff erfolgt in Gegenwart eines Hydrierkatalysators nach den dafür üblichen Techniken kontinuierlich oder diskontinuierlich. Als Katalysatoren eignen sich gängige Hydrierkontakte, z. B. Kontakte, die die Metalle Nickel, Kobalt, Silber, Platin, Palladium und/oder Kupfer

enthalten. Sie können trägerlos, wie beispielsweise Raney-Nickel, Raney-Cobalt oder als Trägerkatalysatoren eingesetzt werden. Die trägerlosen Katalysatoren können auch Verbindungen der genannten Metalle, bevorzugt ihre Oxide sein. Als Träger eignen sich z. B. Kieselsäure, Aluminiumsilikat, Aluminiumoxid oder Kohle. Vorteilhafte Katalysatoren sind Palladium auf Aktivkohle oder Aluminiumoxid in Formstücken, Kupfer auf Aluminiumoxid oder Raney-Nickel. Bei der chargenweisen Durchführung werden dabei 0,05 bis 5,0, vorzugsweise 0,05 bis 2 Gew.% des suspendierten Katalysators, bezogen auf die eingesetzte α, β-Dicarbonylverbindung zugezetzt. Bei der Hydrierung im kontinuierlichen Betrieb wird der Kontakt bevorzugt in stückiger festgebundener Form in einem durchströmten Druckreaktor vorgelegt.

Die Hydrierung wird zweckmäßig bei Temperaturen oberhalb von 30 und unterhalb von 400 °C bei Wasserstoffdrücken von 10 bis 350, vorzugsweise 20 bis 250 bar durchgeführt. Vorteilhaft sind Temperaturen im Bereich von 50 bis 300, insbesondere 120 bis 200 °C.

In der Regel liegen die Reaktionszeiten für die Hydrierung bei 2 bis 5 Stunden. Bei der kontinuierlichen Durchführung entspricht dies dem Kehrwert des Zulaufvolumens in der Zeit, bezogen auf das freie Reaktorvolumen.

Die Isolierung der Imidazole aus den Hydrierausträgen geschieht bei diskontinuierlicher Durchführung in der Regel in der Weise, daß man den Katalysator abtrennt, z. B. durch Filtration und das Reaktionsgemisch gegebenenfalls nach vorheriger Extraktion der Imidazole fraktioniert destilliert. Die Destillation erfolgt nach den dafür üblichen Techniken kontinuierlich oder diskontinuierlich, drucklos oder unter Druck.

Nach dem erfindungsgemäßen Verfahren werden Ausbeutesteigerungen von bis zu 15 % erreicht.

Imidazole und seine Derivate sind wertvolle Zwischenprodukte für Pharmaka, Pflanzenschutzmittel und Farbstoffe.

## Beispiel 1

Eine wäßrige Lösung von 40 %igem Glyoxal, 37 %igem Formaldehyd und 19 %igem Ammoniak wird im Molverhältnis 1 : 1 : 3 bei einer Reaktionstemperatur von 60 °C innerhalb von 3 Stunden umgesetzt.

Die entstandene braune Roh-Imidazol-Lösung wird anschließend destillativ aufgearbeitet, dazu wird zunächst nicht umgesetzter Ammoniak und Wasser bei Normaldruck abdestilliert. Das Imidazol destilliert dann bei 26 mbar und 165 °C über und fällt in einer Ausbeute von 69 % d. Th. an. Der Destillationsrückstand beträgt 38 Gew.% des erhaltenen Imidazols.

## Beispiel 2

Eine gemäß Beispiel 1 hergestellte Lösung von Roh-Imidazol wird zusammen mit 3 Gew.% Raney-Nickel 4 Stunden auf 200 °C bei einem Wasserstoffdruck von 250 bar erhitzt. Danach hat sich die zuvor braune Lösung weitgehend entfärbt und der Katalysator wird abfiltriert. Nach der entsprechend Beispiel 1 durchgeführten Destillation wird jetzt eine Imidazolausbeute von 79 % d. Th. erhalten, hydrierte Imidazole sind nicht nachzuweisen. Der Destillationsrückstand hat sich auf 10 Gew.% des erhaltenen Imidazols, das eine gaschromatographische Reinheit von 99,4 % zeigt, verringert.

## Beispiel 3

Eine gemäß Beispiel 1 hergestellte Lösung von Roh-Imidazol wird in Gegenwart von 0,5 Gew.% eines Katalysators bestehend aus 10 % Palladium auf Aktivkohle 3 Stunden auf 200 °C bei einem Wasserstoffdruck von 30 bar erhitzt.

Die filtrierte farblose Lösung wird wie in Beispiel 1 beschrieben destilliert. Das erhaltene farblose Imidazol zeigt eine gaschromatographische Reinheit von 99,2 % und fällt in einer Ausbeute von 84% d. Th. an. Der Destillationsrückstand hat sich gegenüber Beispiel 1 von 38 auf 9 Gew.% des Reinimidazols verringert.

## Beispiel 4

a) In 2 550 g einer 20 %igen Lösung von Ammoniak in Wasser (30 g Mol) wird in einer Stunde das Gemisch aus 1 450 g (40 %igem wäßrigem) Glyoxal (10 g Mol) und 440 g Acetaldehyd (10 g Mol) unter Rühren eingeleitet. Die Reaktionstemperatur wird durch Kühlung bei 40 °C gehalten. Anschließend wird 2 Stunden auf 80 °C geheizt.

b) Von 2 000 g des nach a) hergestellten Reaktionsproduktes wird bei Normaldruck das Wasser abdestilliert. Bei weiterem Erhitzen und einem Druck von 40 mbar erhält man 314 g 2-Methyl-imidazol, entsprechend 85 % d. Th. Der Destillationsrückstand beträgt 78 g, bezogen auf das erhaltene Methylimidazol sind das 25 Gew.%.

c) 2 000 g des nach a) hergestellten Reaktionsproduktes werden mit 20 g eines Kontaktes aus 20 % Kupfer auf Aluminiumoxid in einem Autoklaven 3 Stunden auf 200 °C erhitzt. Wasserstoff wird dabei bis zu einem Druck von 20 bar aufgepreßt. Nach dem Abtrennen des Kontaktes erhält man bei der

destillativen Aufarbeitung des Hydrierproduktes 340 g Methylimidazol, entsprechend 92 % d. Th. neben 37 g Rückstand, entsprechend 11 Gew.% ; bezogen auf Methylimidazol.

Beispiel 5

Die gemäß Beispiel 1 hergestellte, wäßrige Rohlösung von Imidazol wird in einem Extraktor mit dem gleichen Volumen Isopentanol extrahiert. Der alkoholische Extrakt wird auf zwei Wegen aufgearbeitet :

a) Die destillative Aufarbeitung an einer Claisenapparatur liefert aus 1 800 g Extrakt Isopentanol und 139 g Imidazol von Fp. 88-89 °C und einer gaschromatographischen Reinheit von 99,2 %. Der Destillationsrückstand beträgt 15 g.

b) Dem Extrakt werden 2 % Raney-Nickel zugesetzt. Bei einem Wasserstoffdruck von 30 bar wird er 3 Stunden auf 150 °C gehalten. 1 800 g dieses Hydrierproduktes liefern bei der Destillation 147 g Imidazol vom Fp. 88-89 °C, und einer gaschromatographischen Reinheit von 99,4 %. Der Destillationsrückstand beträgt 7 g.

**Patentansprüche**

1. Verfahren zur Herstellung von Imidazolen durch Umsetzung einer α, β-Dicarbonylverbindung mit einem Aldehyd und Ammoniak, dadurch gekennzeichnet, daß man das nach der Umsetzung anfallende rohe Reaktionsgemisch direkt oder nach Wechsel des Lösungsmittels bei Temperaturen oberhalb von 30 und unterhalb von 400 °C und bei einem Wasserstoffdruck von 10 bis 350 bar katalytisch hydriert.

2. Verfahren nach Anspruch 1, dadurch gekennzeichnet, daß man als α, β-Dicarbonylverbindung Glyoxal verwendet.

3. Verfahren nach Anspruch 1, dadurch gekennzeichnet, daß man Aldehyde der allgemeinen Formel $R^1HC = O$ verwendet, in der $R^1$ einen Alkylrest, ein Arylrest oder vorteilhaft Wasserstoff bedeutet.

4. Verfahren nach Anspruch 1, dadurch gekennzeichnet, daß der Alkylrest aus 1 bis 10 Kohlenstoffatomen besteht und der Arylrest 6 bis 12 Kohlenstoffatome aufweist.

5. Verfahren nach Anspruch 1, dadurch gekennzeichnet, daß die Hydrierung in wäßriger Lösung oder in einem inerten organischen Lösungsmittel wie Alkoholen, Ethern oder tert. Aminen vorgenommen wird.

6. Verfahren nach Anspruch 1, dadurch gekennzeichnet, daß als Hydrierkatalysatoren Nickel-, Kobalt-, Silber-, Palladium-, Platin-, und/oder Kupferkatalysatoren verwendet werden.

**Claims**

1. A process for the preparation of an imidazole by reacting an α, β-dicarbonyl compound with an aldehyde and ammonia, wherein the crude reaction mixture obtained after the reaction is catalytically hydrogenated at above 30 °C and below 400 °C under a hydrogen pressure of from 10 to 350 bar either directly or after the solvent has been replaced.

2. A process as claimed in claim 1, wherein the α, β-dicarbonyl compound used is glyoxal.

3. A process as claimed in claim 1, wherein an aldehyde of the general formula $R^1HC = O$, where $R^1$ is alkyl, aryl or, advantageously, hydrogen, is used.

4. A process as claimed in claim 1, wherein alkyl is of 1 to 10 carbon atoms and aryl is of 6 to 12 carbon atoms.

5. A process as claimed in claim 1, wherein the hydrogenation is carried out in aqueous solution or in an inert organic solvent, such as alcohol, an ether or a tertiary amine.

6. A process as claimed in claim 1, wherein nickel, cobalt, silver, palladium, platinum and/or copper catalysts are used as hydrogenation catalysts.

**Revendications**

1. Procédé de préparation d'imidazoles par réaction d'un dérivé α, β-dicarbonylé avec un aldéhyde et de l'ammoniac, caractérisé par le fait que l'on hydrogène catalytiquement, à des températures supérieures à 30 et inférieures à 400 °C et à une pression d'hydrogène de 10 à 350 bar, le mélange de réaction brut obtenu après la réaction, directement ou après échange du solvant.

2. Procédé selon la revendication 1, caractérisé par le fait qu'on utilise du glyoxal comme dérivé α, β-dicarbonylé.

3. Procédé selon la revendication 1, caractérisé par le fait qu'on utilise des aldéhydes de formule générale $R^1HC = O$, dans laquelle $R^1$ représente un reste alkyle, un reste aryle ou de préférence l'hydrogène.

4. Procédé selon la revendication 1, caractérisé par le fait que le reste alkyle comporte 1 à 10 atomes de carbone et le reste aryle 6 à 12 atomes de carbone.

5. Procédé selon la revendication 1, caractérisé par le fait que l'hydrogénation est effectuée en solution aqueuse ou dans un solvant organique inerte, tels qu'alcools, éthers ou amines tertiaires.

6. Procédé selon la revendication 1, caractérisé par le fait qu'on utilise, comme catalyseurs d'hydrogénation, des catalyseurs au nickel, cobalt, argent, palladium, platine et/ou cuivre.